# EUROPEAN PATENT SPECIFICATION

(11) **EP 4 235 176 B1**
(45) Date of publication and mention of the grant of the patent: **07.05.2025**
(21) Application number: 23170509.6
(22) Date of filing: 14.03.2018
(51) Int. Cl.: G01N 33/543, G01N 33/53

(54) **IMMUNOCHROMATOGRAPHIC DEVICE FOR EXTRACTING AND MEASURING CARBOHYDRATE ANTIGENS**
IMMUNOCHROMATOGRAFISCHE VORRICHTUNG ZUR EXTRAKTION UND MESSUNG VON KOHLENHYDRATANTIGENEN
DISPOSITIF IMMUNOCHROMATOGRAPHIQUE POUR L'EXTRACTION ET LA MESURE D'ANTIGÈNES D'HYDRATE DE CARBON

(30) Priority: 14.03.2017 JP 2017049163
(43) Date of publication of application: 30.08.2023
(62) Divisional of application: 18767950.1
(73) Proprietor: Denka Company Limited, Tokyo 103-8338 (JP)
(72) Inventor: Kato, Daisuke, Niigata, 959-1695 (JP); Muramatsu, Shino, Niigata, 959-1695 (JP); Hattori, Tomohiro, Niigata, 959-1695 (JP)
(74) Representative: Dietz, Christopher Friedrich

(56) References cited:
- EP-A1- 3 032 260
- EP-A1- 3 324 185
- US-A1- 2015 010 918

## Description

### Technical Field

The present invention relates to an immunochromatographic device comprising an immunochromatographic test piece for extracting and measuring a sugar chain antigen, which is capable of extracting the sugar chain antigen with nitrous acid on an immunochromatographic test piece. The invention further relates to a corresponding method.

### Background Art

A majority of rapid diagnostic assayinvolving an immunochromatography method as a principle have been broadly used as a means for promptly and simply measuring viral or bacterial infection and determining a treatment plan therefor.

In the case of such rapid diagnostic assayinvolving a common immunochromatography method as a principle, a specimen is suspended in a specimen suspension, and the suspension is then supplied to an immunochromatographic test piece, so that the measurement can be carried out rapidly and simply.

For detecting microorganisms belonging to genus *Streptococcus*, such as group A β-hemolytic streptococcus and intraoral streptococcus, it is necessary to extract a sugar chain antigen and measure the sugar chain antigen.

For example, a method, in which sodium nitrite and a neutralizing reagent are previously added to an immunochromatographic test piece, so that a nitrous acid extraction can be carried out on the immunochromatographic test piece only by the operation to suspend a specimen in an acid solution such as acetic acid and to supply the suspension to the immunochromatographic test piece, has been reported (as described in the prior art document WO 2005/121794 A1).

Also, there is a method, in which an acid reagent and a neutralizing reagent are previously added to an immunochromatographic test piece, and a specimen is suspended in nitrite and supplied to the immunochromatographic test piece.

For an immunochromatography method, it may be necessary to control a chromatographic development speed on an immunochromatographic test piece, and an immunochromatography method of controlling the development speed on the basis of a device shape, an adhering position, a material to be used (a material such as a non-woven fabric), a reagent, or the like has been reported (as described in the prior art document JP 2005-331471 A and 2005-331463 A).

Prior art document US 2015/0010918 A1 discloses a method for determining the presence or absence of an analyte in a sample with minimal sample preparation. It further provides immunochromatographic devices for determining the presence or absence of an analyte in a sample from which the analyte should be extracted, this device comprising an analyte extraction zone wherein one or more mobile extraction solutions are impregnated, and wherein at least one of said extraction solutions comprises an animal serum or plasma component.

Further, prior art document EP 3 032 260 A1 provides an immunoassay method for detecting the detection target antigen in an analyte through extraction with an extraction agent. The detection is performed in the presence of a cyclic oligosaccharide. An immunochromatography kit is used for detecting gram-positive bacteria in an analyte and provides an analyte dilution solution, a sample dropping part, an antigen extracting part, a labeling substance retaining part, an immunochromatography medium having a detection part, and an absorption part. In the immunochromatography kit an organic acid is retained in the antigen extracting part. The immunochromatography kit contains a cyclic oligosaccharide and a nitrite compound by containing at least one of a cyclic oligosaccharide, a nitrite compound, and a mixture thereof in the analyte dilution solution and/or the sample dropping part.

Still further, prior document EP 3 324 185 A1 describes an immunoassay method that enables an immunoassay with high sensitivity at a high development rate without causing aggregation of insoluble carriers or non-specific reaction while improving test efficiency and reducing labor. The immunoassay method uses a test device and includes: extracting an antigen of a detection target in an analyte with an extraction agent; and detecting the detection target with a detection reagent capable of binding the antigen. The extraction agent is a nitrous acid generated on the test device by a contact reaction between a nitrite salt and a heterocyclic compound having at least one skeleton selected from the group consisting of a cyclic ester, a cyclic amide, and a cyclic imide.

### Summary of the Invention

### Technical Problem

In order to detect microorganisms belonging to genus *Streptococcus*, such as group A β-hemolytic streptococcus and intraoral streptococcus, a sugar chain antigen is extracted, and the sugar chain antigen is measured.

For extracting a sugar chain antigen, nitrous acid is necessary. Since nitrous acid alone is unstable, the extraction of an antigen with nitrous acid is performed by mixing a nitrite solution and an acid solution (acetic acid, citric acid, tartaric acid, or the like) in use to generate the nitrous acid.

In the case of detection by an immunochromatography method, any one of a nitrous acid solution and an acid solution may be applied, dried, and incorporated into an immunochromatographic test piece. Such a reagent, compared with a liquid reagent, eliminates the need of the operation to mix reagents and simplifies operations, whereas a problem of the reagent is insufficient sensitivity because a nitrous acid extraction treatment is not sufficiently performed when a sample is developed in a short time, as in a general immunochromatography method, after reagent mixing.

In a reagent, in which nitrous acid extraction is carried out by adding a liquid reagent, in general, a sample is subjected to extraction for 1 to 2 minutes. Accordingly, for producing sensitivity equivalent to that of a liquid form, it is necessary to retain a sample for 1 to 2 minutes upstream of neutralization.

It is an object of the present invention is to provide a method and an immunochromatographic device, which are capable of measurement with sufficient sensitivity by performing a nitrous acid extraction treatment over a sufficient period of time in an immunochromatography method of extracting and measuring a sugar chain antigen by nitrous acid extraction on an immunochromatographic test piece.

### Technical solution

The object of the invention is realized with a immunochromatic device according to claim 1. Using the immunochromatographic device of the present invention, group A β-hemolytic streptococcus can be detected with high sensitivity.

The inventors of the present invention have conducted intensive studies regarding a technique of performing a nitrous acid extraction treatment over a sufficient period of time in an immunochromatography method for extracting and measuring a sugar chain antigen, which is capable of extracting the sugar chain antigen with nitrous acid on an immunochromatographic test piece.

It was found that the object can be attained by widening an addition port of an immunochromatographic device such that a large amount of specimen can be added, and incorporating thereinto the mechanism to prevent a specimen from flowing out of the addition port and to adjust a development rate, thereby completing the present invention. Specifically, the immunochromatographic device according to the invention comprises an immunochromatographic test piece for extracting and measuring a sugar chain antigen in a specimen, and a container which stores the test piece, the immunochromatographic device having a specimen addition port in a sample pad of the test piece, the immunochromatographic test piece comprising: a sample pad to which a specimen mixed with nitrite or an acid solution is added; a label region comprising a labeled antibody obtained by labeling an antibody against the sugar chain antigen; and a detection region on which the antibody against the sugar chain antigen is immobilized, wherein an antibody-sugar chain antigen-labeled antibody complex is formed in the detection region to measure the sugar chain antigen, and the immunochromatographic test piece having a region impregnated with a neutralizing reagent upstream of the label region, and further having a region impregnated with a solid acid reagent when the specimen mixed with the nitrite is used, or a region impregnated with nitrite when the specimen mixed with the acid solution is used, upstream of the region impregnated with the neutralizing reagent, wherein
the immunochromatographic device
(i) has a specimen addition port for promoting the extraction of the sugar chain antigen with the nitrite and the solid acid reagent by retaining an added specimen sample solution and supplying the specimen sample solution in a short time to the region impregnated with the solid acid reagent or the nitrite, wherein the specimen addition port of the immunochromatographic device has a size to which 10 µL to 200 µL of the specimen sample can be supplied, and
(ii) has no space between the addition port and the sample pad so as to prevent the sample from escaping from the addition port.

Such a immunochromatographic device has the advantage that a development time of a specimen sample solution on an immunochromatographic test piece after sample addition can be adjusted. As a result, a specimen can be sufficiently treated on the test piece by use of a specimen treatment reagent added onto the immunochromatographic test piece.

Advantageous embodiments of the invention are defined by the dependent claims.

The invention further relates to a method of measuring a sugar chain antigen in a specimen according to an immunochromatography method using the immunochromatographic device according to claim 14.

### Brief Description of the Drawings

Figure 1 is a view schematically showing a structure of an immunochromatographic test piece (one-pad test piece) having a solid acid reagent region and a neutralizing reagent region.
Figure 2 is a view schematically showing a structure of an immunochromatographic test piece (two-pad test piece) having a solid acid reagent region and a neutralizing reagent region.
Figure 3 is a perspective view showing an outer appearance of an immunochromatographic device. Figure 3A shows the device of the present invention having a wide addition port. Figure 3B shows a conventional device having a narrow addition port.
Figure 4 is a plane view showing an outer appearance of an immunochromatographic device. Figure 4A shows the device of the present invention having the wide addition port. Figure 4B shows a conventional device having the narrow addition port.

### Description of Embodiments

The immunochromatographic device simplifies a treatment of extracting a sugar chain antigen with nitrous acid on an immunochromatographic test piece, so that the sugar chain antigen used as a substance to be detected can be measured promptly and accurately. The immunochromatographic device comprises an immunochromatographic test piece and a container which stores the test piece, and can be produced by incorporating the immunochromatographic test piece into the immunochromatographic device. Such a storing container can prevent the degradation of the test piece, which is caused by, for example, ultraviolet ray or moisture contained in the air. Moreover, in the case of treating a specimen or a sample having contamination or infectivity, such a storing container can prevent a tester performing an assay from being contaminated or infected with the specimen or the sample. For instance, a resin-made case having a suitable size may be used as a storing container, and the immunochromatographic device may be accommodated in the case. Otherwise, the surface of a test piece, on which an antigen or an antibody has been immobilized, may be coated with a resin-made film or the like (top laminate).

The immunochromatographic test piece comprises a support having a detection region, on which an antibody (Antibody 1) that captures a substance to be detected (antigen, etc.) is immobilized, a label region having a movable labeled antibody (Antibody 2), a sample pad to which a specimen is added, an absorption band that absorbs a developed specimen solution, a backing sheet for adhering these members to one another, and the like.

It is to be noted that the number of detection regions and the type of a labeled antibody contained in the label region are not limited to one, and that, by using antibodies corresponding to a plurality of substances to be detected, two or more antigens can be measured on a single test piece.

The support is a material having the property of immobilizing an antibody used to capture a substance to be detected (an antigen), and also, it does not prevent the movement of a liquid in the horizontal direction. Preferably, the support is a porous thin membrane (a membrane) having capillary action, and is a material capable of transporting a liquid and components dispersed in the liquid according to absorption. The material used for the support is not particularly limited, and examples of the material include cellulose, nitrocellulose, cellulose acetate, polyvinylidene difluoride (PVDF), glass fiber, nylon, and polyketone. Among these materials, a thin membrane or a membrane of nitrocellulose is more preferable. A membrane, on which an antibody is immobilized, is referred to as an "antibody-immobilized membrane."

The label region consists of a porous substrate comprising a labeled antibody, and a commonly used glass fiber, non-woven fabric, and the like can be used herein as a material for the substrate. The substrate is preferably a pad having a thickness of approximately 0.3 mm to 0.6 mm, in order that the substrate is impregnated with a large amount of labeled antibody. A porous substrate that is impregnated with a labeled antibody and is then dried is also referred to as a dry pad.

For the labeling of a labeled antibody, enzymes such as alkaline phosphatase or horse radish peroxidase, metal colloids such as gold colloids, silica particles, cellulose particles, colored polystyrene particles, colored latex particles, etc. are used in many cases. When metal colloidal particles or colored particles such as colored polystyrene particles or colored latex particles are used, color is developed by clumping of these labeling reagents. So, the thus developed color is measured. Particles, on which antibodies are immobilized, are referred to as antibody-immobilized particles.

The detection region indicates a region of the support, on which an antibody used to capture a substance to be detected (an antigen) is immobilized. In the detection region, at least one region, on which an antibody used to capture an antigen is immobilized, is established. The detection region may be comprised in the support, and an antibody may be immobilized on the support.

The sample pad is a site to which a specimen is added, and is a porous material. The sample pad is a site located most upstream of the immunochromatographic test piece. As a material for the sample pad, a commonly used filter, glass fiber, non-woven fabric, etc. can be used. In order to use a large amount of specimen in immunoassay, the sample pad is preferably a pad having a thickness of approximately 0.3 mm to 1 mm. The specimen also includes a sample prepared using the specimen, such as a sample obtained by suspending the specimen in another solution.

The absorption band is a member for absorbing components, which are supplied to the support and are not associated with the reaction in the detection region. As a material for the absorption band, a highly water-retainable filter, sponge or the like, consisting of a common natural high-molecular-weight compound, synthetic high-molecular-weight compound, etc. can be used. In order to promote the development of a specimen, highly water-absorbable material is preferably used as an absorption band.

The backing sheet is a member used for adhesion and/or immobilization of all of the aforementioned materials, namely, the support, the sample pad, the label region, the absorption band and the like, in which these materials are partially overlapped with one another. The backing sheet is not necessarily required, if these materials are arranged and immobilized with optimal intervals. However, in general, the backing sheet is preferably used for convenience or ease of production or use.

In the immunochromatographic test piece, a control display region (a member) may be further present. The control display region is a site showing that a test is accurately carried out. For example, the control display region is located downstream of the detection region, and emits signals such as coloration, when a specimen sample is passed through the detection region and reaches the control display region. On the control display region, a substance binding to a labeled carrier-bound antibody may be solid-phased, or a reagent such as a pH indicator, the color of which is changed when a specimen reaches, may also be solid-phased. When such a labeled carrier-bound antibody is a mouse monoclonal antibody, an anti-mouse IgG antibody may be used.

The size of an immunochromatographic test piece is not limited. For example, the height thereof is from several cm to more than ten and less than 20 cm, and the width thereof is from approximately several mm to several cm.

In the test piece having the above-described form, the specimen is passed through a porous flow channel formed by connecting a series of members, such as the sample pad, the label region, the support, the detection region, the absorption band and the like, with one another. Accordingly, in the present embodiment, all of these components constitute a specimen moving region. There may also be an embodiment, in which the specimen does not penetrate into various constitutional materials but pass through the interface, depending on the materials or forms of the constitutional materials. However, since the specimen moving region defined in the present description is irrelevant to whether the specimen passes into the material or passes through the interface, the test piece having the aforementioned embodiment is also included in the scope of the present description.

In the case of measuring a sugar chain antigen in a specimen using the immunochromatographic test piece, it is necessary to first extract the sugar chain antigen in the specimen. The extraction of the sugar chain antigen is performed by treating the specimen containing the sugar chain antigen with nitrous acid. The nitrous acid can be generated by mixing nitrite such as sodium nitrite with an acid, and the specimen containing the sugar chain antigen may be treated with the nitrous acid thus generated. The extracted antigen binds through an antigen-antibody reaction to the antibody immobilized on the immunochromatographic test piece. In this respect, the reaction system becomes strongly acidic if nitrous acid remains in the reaction system, so that the antigen-antibody reaction is inhibited. Thus, it is necessary to neutralize nitrous acid in the reaction system.

In the method of measuring a sugar chain antigen by mixing nitrite with an acid to generate nitrous acid, extracting the sugar chain antigen from a specimen with the nitrous acid, neutralizing the nitrous acid, and then allowing the sugar chain antigen to bind to the antibody immobilized on the immunochromatographic test piece, examples of the method of extracting and measuring the sugar chain antigen include the following methods. In any of the methods, the extraction of the sugar chain antigen with nitrous acid, and the neutralization are performed on the immunochromatographic test piece. In order to perform the extraction of the sugar chain antigen with nitrous acid on the immunochromatographic test piece, the immunochromatographic test piece can be impregnated with an acid reagent or nitrite. In order to perform the neutralization on the immunochromatographic test piece, the immunochromatographic test piece can be impregnated with a neutralizing reagent.
(A) A specimen is previously mixed with an acid solution, and the mixture is added to a sample pad of the immunochromatographic test piece impregnated with nitrite and a neutralizing reagent. When the mixed solution arrives at the region impregnated with the nitrite, the nitrite reacts with the acid to generate nitrous acid, so that a sugar chain antigen in the specimen is extracted. The extract of the sugar chain antigen is neutralized in the region impregnated with the neutralizing reagent on the immunochromatographic test piece, and the sugar chain antigen binds to the antibody immobilized on the immunochromatographic test piece and can thus be detected. In this method, examples of the acid solution used include acetic acid, hydrochloric acid, malonic acid, malic acid, maleic acid, citric acid, and tartaric acid.
(B) A specimen is previously mixed with a nitrite solution, and the mixture is added to a sample pad of the immunochromatographic test piece impregnated with an acid reagent and a neutralizing reagent. When the mixed solution arrives at the region impregnated with the acid reagent, the nitrite reacts with the acid to generate nitrous acid, so that a sugar chain antigen in the specimen is extracted. The extract of the sugar chain antigen is neutralized in the region impregnated with the neutralizing reagent on the neutralizing immunochromatographic test piece, and the sugar chain antigen binds to the antibody immobilized on the immunochromatographic test piece and can thus be detected.

In the immunochromatographic test piece for performing the above-described method (A) or (B), the acid reagent or the nitrate and the neutralizing reagent are impregnated upstream of the label region (on the upstream side along the flow of the specimen, where the sample pad is present), namely, within the sample pad or between the sample pad and the label region. The immunochromatographic test piece, in which the nitrite and the neutralizing reagent are impregnated within the sample pad or between the sample pad and the label region, can be used in the above-described method (A). Also, the immunochromatographic test piece, in which the acid reagent and the neutralizing reagent are impregnated within the sample pad or between the sample pad and the label region, can be used in the above-described method (B). It is to be noted that as the acid reagent to be impregnated into the immunochromatographic test piece, a solid acid reagent is used. According to these methods, a substance to be detected in a test sample can be measured accurately and specifically, regardless of the amount of the test sample subjected to a test.

The solid acid reagent or the nitrite may be impregnated into the sample pad, or may be impregnated into a pad made of a porous material that is different from the sample pad, such as a non-woven fabric, and the obtained solid acid reagent-impregnated porous material or nitrite-impregnated porous material may be disposed between the sample pad and the label region, namely, on the side upstream of the label region. Herein, the region impregnated with the solid acid reagent or the nitrite may be contacted or may not be contacted with the sample pad or the label region.

The region impregnated with a reagent is also referred to as a pad impregnated with a reagent.

The neutralizing reagent is disposed downstream of the region impregnated with the solid acid reagent or the nitrite. The neutralizing reagent may be impregnated into the sample pad, or may be impregnated into the support, or may be impregnated into a pad made of a porous material that is different from the support, such as a non-woven fabric, and the obtained neutralizing reagent-impregnated porous material may be disposed between the region impregnated with the solid acid reagent or the nitrite and the label region. That is, the immunochromatographic test piece has a region impregnated with a neutralizing reagent upstream of the label region, and further has a region impregnated with a solid acid reagent or nitrite upstream of the region impregnated with the neutralizing reagent. Herein, the region impregnated with the neutralizing reagent may be contacted or may not be contacted with the region impregnated with the solid acid reagent or the nitrite or the label region.

The region impregnated with the solid acid reagent is referred to as a solid acid reagent region, the region impregnated with the nitrite is referred to as a nitrite region, and the region impregnated with the neutralizing reagent is referred to as a neutralizing reagent region or a basic reagent region.

The immunochromatographic test piece having a solid acid reagent region or a nitrite region and a neutralizing reagent region has, on the support, a sample pad, a solid acid reagent region or a nitrite region, a neutralizing reagent region, a label region, a detection region, and an absorption band, from the upstream thereof, and the solid acid reagent region or the nitrite region may be located on the sample pad. Moreover, the sample pad, the solid acid reagent region or the nitrite region, the neutralizing reagent region, the label region, the detection region, and the absorption band may be contacted or may not be contacted with a region adjacent thereto. Furthermore, the solid acid reagent region or the nitrite region, the neutralizing reagent region, and the label region are not necessarily impregnated into each different porous material, and a plurality of or all of the regions may be impregnated into a single porous material.

The solid acid reagent used for the immunochromatographic test piece is in a solid state at normal temperature and does not volatilize at a high temperature.

Examples of the solid acid reagent that is preferably can include malonic acid, malic acid, maleic acid, citric acid, and tartaric acid.

If an acid with a higher valence, for example, citric acid, is used as a preferred solid acid reagent, extraction can be performed with a smaller amount of acid. On the other hand, if the valence of an acid is the same, for example, maleic acid or tartaric acid having a smaller acid dissociation constant is more efficient.

Also, the solid acid reagent is preferably a reagent that is not colored on the immunochromatographic test piece, and more specifically, a reagent, which has white color in a dry state or is hardly colored by dry heat or oxidation.

Examples of the nitrite used in the present invention include sodium nitrite and potassium nitrite.

The amount of the solid acid reagent or the nitrite, namely, the amount of the solid acid reagent or the nitrite impregnated into the immunochromatographic test piece is not particularly limited. In general, the amount of the solid acid reagent or the nitrite is approximately 0.01 µg to 1 mg, and is preferably approximately 0.1 µg to 0.1 mg, with respect to a single immunochromatographic test piece. However, it is preferable to select an optimal amount, in which effects can be obtained depending on the type of the solid acid reagent or the nitrite to be used, the composition of a specimen suspension, the amount added, etc.

In order to impregnate a sample pad or a porous material with the solid acid reagent or the nitrite, the solid acid reagent or the nitrite is once dissolved in a solution, and the obtained solution is then applied to the sample pad or the porous material and is then dried.

The neutralizing reagent is in a solid state at normal temperature and does not volatilize at a high temperature.

Examples of the neutralizing reagent that is preferably include Tris base (tris(hydroxymethyl)aminomethane), sodium hydroxide, dipotassium hydrogen phosphate, trisodium citrate, and a Good's buffer having a buffering ability in the alkaline range.

The amount of the neutralizing reagent, namely, the amount of the neutralizing reagent impregnated into the immunochromatographic test piece is not particularly limited. In general, the neutralizing reagent is used in an amount of approximately 0.01 µg to 1 mg, and preferably approximately 0.1 µg to 0.1 mg, for a single immunochromatographic test piece. However, it is preferable to select an optimal amount, in which effects can be obtained depending on the type of the used neutralizing reagent, the composition of a specimen suspension, the amount added, etc.

In order to impregnate a sample pad or a porous material with the neutralizing reagent, the neutralizing reagent may be once dissolved in a solution, the obtained solution may be then applied to the sample pad or the porous material, and thereafter, it may be dried.

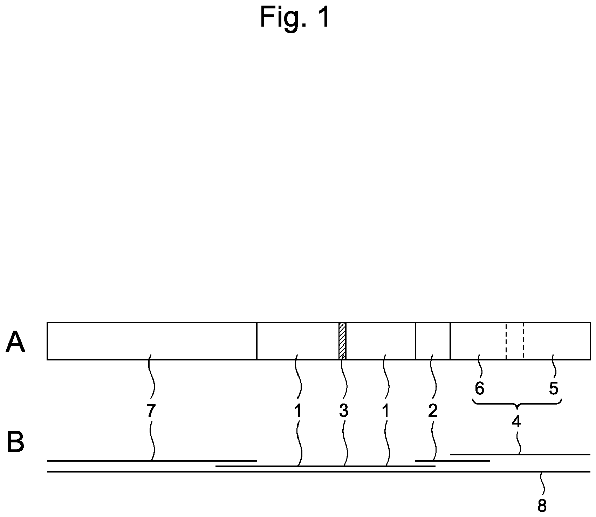

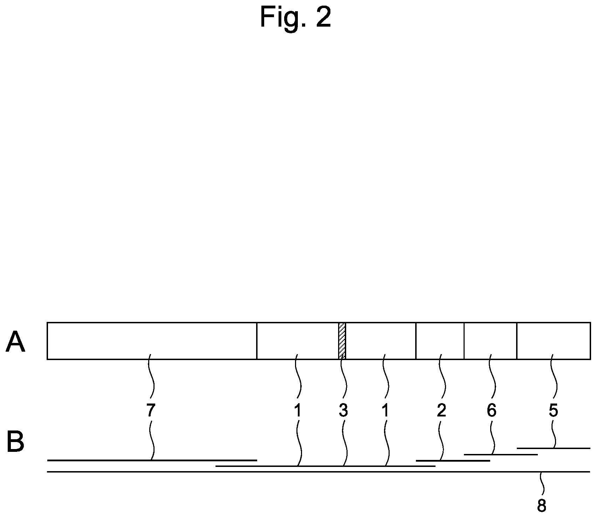

Figure 1 and Figure 2 are views each showing a preferred embodiment of a typical immunochromatographic test piece. The immunochromatographic test piece shown in Figure 1 or Figure 2 is an immunochromatographic test piece impregnated with a solid acid reagent and a neutralizing reagent. However, nitrate may be impregnated instead of the solid acid reagent, and the resultant is an immunochromatographic test piece impregnated with nitrate and a neutralizing reagent. Those skilled in the art can appropriately design and produce an immunochromatographic test piece impregnated with nitrate and a neutralizing reagent. It is to be noted that the immunochromatographic test piece is not limited to those shown in Figure 1 and Figure 2. In Figure 1 and Figure 2, reference numeral 1 denotes a support, reference numeral 2 denotes a label region, reference numeral 3 denotes a detection region, reference numeral 4 denotes a sample pad, reference numeral 7 denotes an absorption band, and reference numeral 8 denotes a backing sheet. Moreover, a top laminate may adhere onto the entire test piece.

Figure 1A and Figure 2A are top views, and Figure 1B and Figure 2B are cross-sectional views. In the example shown in Figure 1, a support 1 on which one detection region 3 is formed, an absorption band 7, a label region 2, a sample pad 4, and the like, are laminated on a backing sheet 8 made of resin or the like. As shown in Figure 1, one end of the absorption band 7 is laminated on one end of the support 1, the other end of the support 1 is laminated on one end of the label region 2, and the other end of the label region 2 is laminated on one end of the sample pad 4. A portion upstream of the sample pad 4 is impregnated with a solid acid reagent, and a portion downstream of the sample pad is impregnated with a neutralizing reagent, which is somewhat apart from the upstream portion. The region impregnated with the solid acid reagent is referred to as a solid acid reagent region 5, and the region impregnated with the neutralizing reagent is referred to as a neutralizing reagent region 6. In this test piece, the sample pad also serves as a solid acid reagent region 5 and a neutralizing reagent region 6. That is, the solid acid reagent region and the neutralizing reagent region are present on the sample pad. This test piece, in which the solid acid reagent region and the neutralizing reagent region are established as one porous material (pad), is also referred to as a one-pad test piece. In the example shown in Figure 2, the solid acid reagent region 5 and/or the neutralizing reagent region 6 is present upstream of the label region 2, and these are overlapped with each other, so that a flow channel of a continuous lateral flow is formed. In the test piece shown in Figure 2, the solid acid reagent region 5 also serves as a sample pad. That is, the solid acid reagent region is present on the sample pad. In this test piece, in which the solid acid reagent region and the neutralizing reagent region are established as two different porous materials (pads), is also referred to as a two-pad test piece. A sample pad may further be present upstream of the solid acid reagent region. In the test piece shown in Figure 2, the solid acid reagent region 5 and the neutralizing reagent region 6 are impregnated into different porous materials. However, on a single porous material (pad), the solid acid reagent region 5 is established in an upstream portion, and the neutralizing reagent region 6 is established in a downstream portion, as in the sample pad of the test piece shown in Figure 1.

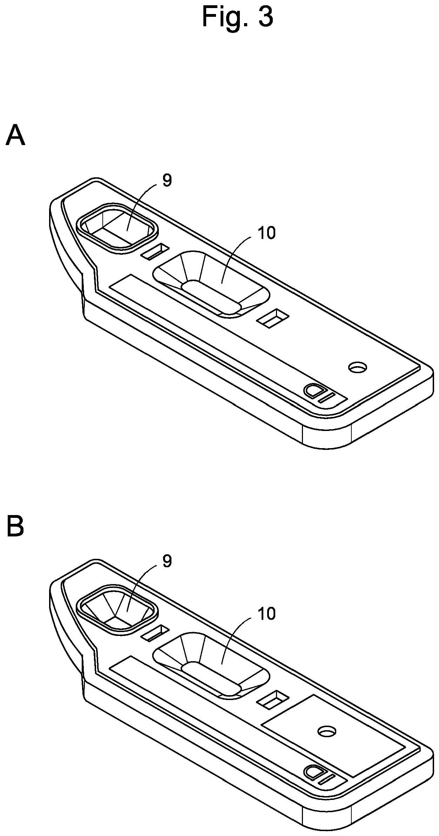

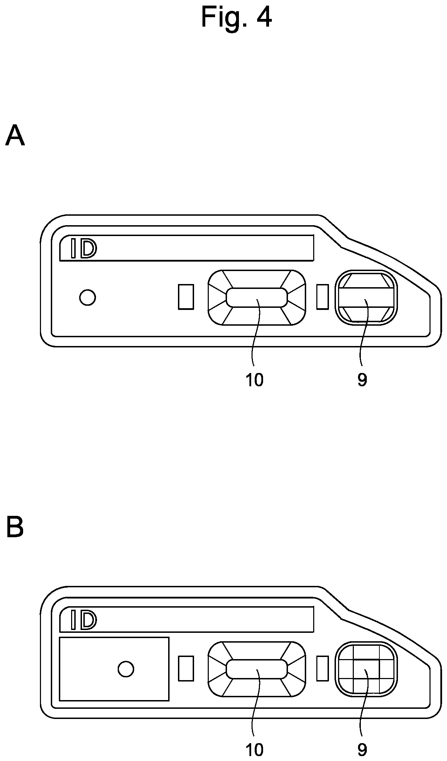

The above-described chromatographic test piece is accommodated in a storing container shown in Figures 3 and 4, and used as an immunochromatographic device (Figures 3 and 4). The storing container shown in Figures 3 and 4 comprises a container portion as a lower portion and a lid portion as an upper portion. The storing container has, as shown in Figures 3 and 4, a specimen addition port (also referred to as an addition hole) 9 and a judgment portion 10. When the above-described chromatographic test piece is accommodated in the storing container, a specimen addition port which can supply a specimen sample solution to the sample pad is positioned above the sample pad. When a specimen is added to the specimen addition port of the container, the specimen infiltrates into the sample pad. When the chromatographic test piece is accommodated in the storing container, the judgment portion is positioned above the detection region, so that the detection region can be observed through the hole of the judgment portion of the container.

The immunochromatographic device can perform a nitrous acid extraction treatment over a sufficient period of time in an immunochromatography method for extracting and measuring a sugar chain antigen, which is capable of extracting the sugar chain antigen with nitrous acid on the immunochromatographic test piece. The immunochromatographic device therefore has the following structural features. The following structural features are structural features regarding the mechanism to adjust the development rate of a specimen sample.
(1) In the immunochromatographic device, a wide addition port is established.

For example, the addition port of a conventional immunochromatographic device (QuickNavi(TM)-Strep A, Denka Seiken Co., Ltd.), in which an immunochromatographic test piece having a length of 5 to 9 cm, preferably 7 cm, and a width of 0.3 to 0.7 cm, preferably 0.5 cm, is stored, is established as a substantially rectangular addition port having a size of approximately 3.5 mm × approximately 5.5 mm (19.25 mm²). The addition port of the immunochromatographic device, in which the immunochromatographic test piece having the same size as above is stored, is established as a substantially rectangular addition port having a size of approximately 3.5 mm × approximately 11 mm (38.5 mm²). Since the immunochromatographic device and the immunochromatographic test piece stored therein generally have similar sizes, an immunochromatographic device of 3 cm wide and 9 cm long having a substantially rectangular addition port of 3 to 5 mm × 8 to 15 mm (24 to 75 mm²) is an immunochromatographic device having a wide addition port and is capable of exerting the effects of the immunochromatographic device. The longer sides of the addition port are sides parallel to the longer sides of the chromatographic test piece, namely, sides along the flow direction of a specimen sample solution. The length of the longer side of the addition port is also referred to as an addition port length, and the length of the shorter side thereof is also referred to as an addition port width. In the above-described example, a substantially rectangular addition port is used. However, the shape of the addition port may be a triangular or round shape. When the size of the addition port of the immunochromatographic device is indicated by area, the area is 24 to 75 mm², preferably 35 to 75 mm².

When a specimen is treated with a specimen treatment reagent established on an immunochromatographic test piece according to a conventional immunochromatography method, specimen-treating ability is weak due to a small area in which a sample solution is contacted with a region impregnated with the specimen treatment reagent. Furthermore, the specimen treatment is performed while the immunochromatographic test piece is gradually developed. Hence, a sample solution developed first and a sample solution developed later differ in the concentration of the specimen treatment reagent. Accordingly, the specimen treatment cannot be performed reliably.

The addition port of the immunochromatographic device is established as a wide addition port and can therefore supply a large amount of specimen sample solution in a short time at once to a region impregnated with a specimen treatment reagent, namely, a region impregnated with a solid acid reagent or a region impregnated with a nitrous acid, upon immunochromatography. As a result, the extraction of the sugar chain antigen on the immunochromatographic test piece can be promoted. The amount of the specimen sample solution that can be supplied at once is 10 µL to 200 µL.

As a result, the difference in the concentration of the specimen treatment reagent caused by temporal difference does not occur. Hence, the specimen treatment upon immunochromatography can be promoted and performed reliably and efficiently.

(2) The immunochromatographic device is configured to have no space between the addition port and the sample pad positioned therebeneath so as to prevent a sample from escaping from the addition port.

The addition port of the immunochromatographic device is established as a wide addition port and supplies a large amount of specimen sample solution in a short time at once. The edge portion, which is an outline, of an addition port has a given height (1 to 5 mm), and a supplied specimen sample solution is temporarily kept within the addition port.

As a result, it takes long for the specimen sample solution to be entirely absorbed to an immunochromatographic test piece, so that the sample solution may be escaped to the outside of the immunochromatographic test piece without being absorbed to the immunochromatographic test piece.

In the immunochromatographic device, the sample pad is supported by a backing sheet having an outline size equivalent to or larger than that of the addition port, as a backing sheet supporting the sample pad, so as to prevent a sample from escaping from the addition port. Thus, the backing sheet can work to press the sample pad against the addition port to clear the space between the addition port and the sample pad. The immunochromatographic device thus configured can prevent a sample solution from escaping.

If the sample pad has an uneven thickness, a thin portion of the sample pad easily forms a space from the addition port and easily causes a sample solution to escape.

For the immunochromatographic device, a pedestal for the container of the device can be designed as a high pedestal for the thin portion of the sample pad and designed as a low pedestal for the thick portion to eliminate the space between the addition port and the pad even having an uneven thickness. The immunochromatographic device thus configured can prevent a sample solution from escaping.

As a result, the extraction of the sugar chain antigen on the immunochromatographic test piece can be carried out efficiently.

(3) Preferably, a highly hydrophobic material is selected as a material (non-woven fabric) for use in the production of a pad impregnated with a specimen treatment reagent, such as a solid acid reagent, nitrite, or a neutralizing reagent, for the immunochromatographic test piece, and can thus adjust and slow a liquid flow speed, namely, the time for which a specimen sample solution is developed on the immunochromatographic test piece.

In this case, examples of the highly hydrophobic material include polyethylene, polyester, polystyrene, polypropylene, rayon, and nylon.

Examples of the porous material to be used in the region impregnated with the neutralizing reagent include a porous material that is a woven fabric having a basis weight of 10 to 400 g/m² and a thickness of 0.1 to 2.0 mm, absorbs water in an amount of 10 to 100 µl/cm² per cm/m², has a water absorption speed of 1.0 to 5.0 µl/sec, retains water in an amount of 10 to 100 µl/cm² after a fragment of 1 cm/m² is allowed to come in a wet state into contact with a membrane and left standing for 5 minutes, and preferably has a liquid spread area of 20 mm² or smaller after a fragment of 1 cm/m² is allowed to come in a wet state into contact with a membrane and left standing for 5 minutes, namely has three properties of being highly water-absorbable, being highly water-retainable (liquid-retainable), and being low liquid-releasable or continuously liquid-releasable. Specific examples thereof include a filter made of cellulose cotton fiber, and a glass filter made of glass fiber. Examples of such a filter include No. 26-3 from Toyo Roshi Kaisha, Ltd. Moreover, the filter to be used has a large capacity and can sufficiently retain an acid solution that reaches the filter later than a specimen from the upstream portion. By using such a porous material, the neutralizing reagent can be impregnated thereinto in an amount that can sufficiently neutralize a specimen from which a sugar chain antigen has been extracted with nitrous acid generated through a reaction of nitrite with the acid reagent. Moreover, the pad can absorb and retain a large amount of liquid and sustains the releasable property. Hence, if a liquid containing nitrous acid remaining upstream of the immunochromatographic test piece is developed at or after the time of judgment, the pad can have a sufficient neutralizing ability and can prevent the acid solution from arriving at the detection region on which the antibody is immobilized. As a result, a non-specific reaction is suppressed, and the sugar chain antigen can be detected without causing the non-specific reaction. On the other hand, specific examples of the highly water-absorbable, highly water-retainable, and low releasable glass filter include GS-25 from Toyo Roshi Kaisha, Ltd. which can be impregnated with a larger amount of neutralizing reagent because of the high water-absorbable property, and prevents the acid solution from arriving at the immobilized detection region because of the high water-retainable property and the low water-releasable property. As a result, a non-specific reaction can be suppressed in a negative case, and color development of a line can be prevented at or after the time of judgment in a positive case.

The "basis weight" of the porous material to be used in the region impregnated with the neutralizing reagent is 10 to 400 g/m². Herein, the "basis weight" indicates a weight per unit area (1 m²) of a fabric or the like. The basis weight can be appropriately changed by adjusting the amount or composition of the neutralizing reagent impregnated into the pad. If the basis weight is 30 g/m² or less, the porous material has a high porosity and is easily torn when impregnated with the neutralizing reagent, and is thus difficult to handle during immunochromatographic production. Hence, the basis weight is preferably 50 g/m² or more. If the basis weight is 300 g/m² or more, the porous material has a low porosity and does not permit smooth penetration of a specimen into the material, albeit depending on the composition of the neutralizing reagent, so that the specimen cannot be mixed with the neutralizing reagent. Hence, the basis weight is preferably 300 g/m² or less. The basis weight is most preferably 250 to 270 g/m².

The "thickness" of the porous material to be used in the region impregnated with the neutralizing reagent is preferably 0.1 to 2.0 mm. The thickness can be appropriately changed by adjusting the amount or composition of the neutralizing reagent impregnated into the pad. If the thickness is 0.4 mm or less, the porous material is easily torn when impregnated with the neutralizing reagent, and is thus difficult to handle during immunochromatographic production. Hence, the thickness is preferably 0.4 to 0.8 mm and is more preferably approximately 0.6 mm in consideration of the easiness with which the amount of the neutralizing reagent impregnated is adjusted, and the easiness of handling during immunochromatographic production.

The "water-absorbable" property is preferably 10 to 100 µl/cm² in terms of an amount of water absorbed per cm/m², and 1.0 to 5.0 µl/sec in terms of a water absorption speed. The amount of water absorbed and the water absorption speed are determined by preparing 200 µl of a colored solution (1 % Tween 20 + Red No. 102) in one cell of a 96-well EIA plate, placing therein a test piece, in which a material having a size of 5 × 60 mm adheres to a backing sheet, measuring the time for the solution to arrive at the upper end of the test piece (an end of the test piece dipped in the solution is defined as a lower end), further taking the test piece out of the solution immediately after the solution reaches the upper end, and measuring the amount of a liquid remaining in the cell. The amount of water absorbed is the amount of a liquid calculated by subtracting the amount of the liquid remaining in the cell from 200 µl, and dividing the obtained value by 1 cm² of the area of the material. The water absorption speed is determined according to the amount of water absorbed / the time required to reach the upper end. The porous material to be used in the region impregnated with the neutralizing reagent is preferably a material having a larger amount of water absorbed and a slower water absorption speed. If the amount of water absorbed is 30 µl/cm² or less, albeit depending on the concentration and water content of the solid acid reagent, it is considered that the amount of the neutralizing reagent impregnated may fall short of an amount necessary for the immunochromatographic test piece. Specifically, the amount of water absorbed is preferably 30 µl/cm² or more. The water absorption speed of the porous material to be used in the region impregnated with the neutralizing reagent influences the time required to extract a sugar chain antigen from a specimen, and the time required to neutralize a test solution after the extraction. The time required to extract a sugar chain antigen can be adjusted to some extent, but not completely, by adjusting the material or composition of the solid acid reagent. Hence, the water absorption speed of the porous material to be used in the region impregnated with the neutralizing reagent is an important factor for sufficient extraction of the sugar chain antigen and neutralization. Specifically, the water absorption speed is preferably 1.0 to 5.0 µl/sec. The water absorption speed is more preferably 2.0 µl/sec or less.

The "water-retainable" property is determined by placing, on a membrane, a fragment of 1 cm/m² obtained from the porous material to be used in the region impregnated with the neutralizing reagent, adding thereto 70 µl of a solution (1 % Tween 20 + Red No. 102), and measuring the weight of the membrane before and after the membrane is left standing for 5 minutes. The amount of a liquid for the water-retainable property varies depending on the composition (a surfactant or a protein) of the solution to be added. In the case of a test using a 1 % Tween 20 solution, the amount of water retained is preferably 10 to 100 µl/cm². The amount of water retained is more preferably 15 µl/cm² or more.

The "releasable" property is measured by placing, on a membrane, a fragment of 1 cm/m² obtained from the porous material to be used in the region impregnated with the neutralizing reagent, adding thereto 70 µl of a solution (1 % Tween 20 + Red No. 102), and determining the area of a liquid spread on the membrane after the membrane is left standing for 5 minutes. The area varies depending on the composition (a surfactant or a protein) of the solution to be added. In the case of a test using a 1 % Tween 20 solution, the area is 30 mm² or less. The releasable property is more preferably 20 mm² or less as this area.

The reagent to be added as a specimen treatment reagent, such as a solid acid reagent, nitrite, or a neutralizing reagent, to a highly hydrophobic pad is further supplemented with a surfactant including: polyoxyethylene octyl phenyl ether such as Triton X-100 and Triton X-114; polyoxyethylene sorbitan fatty acid ester such as Tween 20 and Tween 80; polyoxyethylene alkyl ether such as Brij-35 and Brij-58; a cholic acid-based surfactant having a steroid skeleton, such as sodium cholate and deoxycholic acid; and an anionic surfactant such as sodium lauryl sulfate and sodium dodecylbenzenesulfonate, so that the degree of hydrophobicity of the reagent is controlled. Thus, a specimen treatment time on the addition port can be set to any time. Preferably, by adding the surfactant, a specimen can be treated for a sufficiently period of time, and a specimen sample solution can be properly developed. That is, the specimen can be sufficiently treated with the solid acid reagent or the nitrite to extract a sugar chain antigen from the specimen. Furthermore, the resultant can be developed to the downstream portion having the pad impregnated with the neutralizing reagent.

A material having a high degree of hydrophilicity is selected as a material for use in the production of a pad impregnated with a specimen treatment reagent, such as a solid acid reagent, nitrite, or a neutralizing reagent, for the immunochromatographic test piece, and can thus adjust and slow a liquid flow speed, namely, the time for which a specimen sample solution is developed on the immunochromatographic test piece, instead of controlling the liquid flow speed by use of the highly hydrophobic pad and the surfactant. Examples of the material having a high degree of hydrophilicity include a thick filter, and the thickness thereof is 210 to 580 µm.

Instead of the surfactant, another high-molecular-weight compound having immobilizing action (e.g., PVA (polyvinyl alcohol) and PLL (poly-L-lysine) may be used such that a sample solution is easily retained on the pad. In this method as well, the extraction of the sugar chain antigen on the immunochromatographic test piece can be promoted.

For the immunochromatographic device, the time for which an added specimen sample solution is developed on the immunochromatographic test piece is lengthened, so that development to the downstream pad impregnated with the neutralizing reagent (the neutralizing reagent region) can be delayed.

The immunochromatographic device can be utilized not only as a device for extracting a sugar chain antigen with nitrous acid on the immunochromatographic test piece, but also as an immunochromatographic device for treating a specimen on the immunochromatographic test piece with a reagent impregnated on the immunochromatographic test piece.

The immunochromatographic device can be utilized when an analyte to be detected is incubated in a test device of an immunochromatographic reagent, depending on pH or a compound, in addition to extraction with nitrous acid.

A method of using the immunochromatographic device will be described on the basis of an immunochromatographic device, in which the immunochromatographic test piece having the form shown in Figure 1 is stored in the storing container shown in Figure 4. The following use method is a method of mixing a specimen with a nitrous acid solution and carrying out measurement by use of an immunochromatography method, in which a solid acid reagent and a neutralizing reagent are impregnated. A method of mixing a specimen with an acid solution and carrying out measurement by use of an immunochromatography testing method, in which nitrite and a neutralizing reagent are impregnated, can also be performed with reference to the description of the following use method.

A specimen or a sample prepared using the specimen is contacted and mixed with a nitrite solution, and the specimen is suspended in the nitrite solution, and the suspension is then added to the specimen addition port of the device, so that the measurement is initiated. This time, 5 to 100 µL of a specimen may be mixed with 0.01 to 2 mL of 0.1 M to 8 M nitrite, and 5 to 200 µL of the mixture may be then added to the addition port. Examples of the nitrite include sodium nitrite and potassium nitrite.

A specimen containing a sugar chain antigen as a substance to be detected, which is subjected to the addition port 9, is moved to a sample pad and developed to a solid acid reagent region 5 and a neutralizing reagent region 6 on the sample pad 4 according to capillary action, and further, is successively developed to a label region 2, a support 1, and an absorption band 7 in the horizontal direction. In the solid acid reagent region 5, nitrite mixed with the specimen reacts with a solid acid reagent on the solid acid reagent region 5 to generate free nitrous acid, so that a sugar chain antigen is extracted from the specimen by the action of the nitrous acid. The extracted sugar chain antigen is developed and moved, together with an acidic developing solution, to the neutralizing reagent region 6, and the pH of the acidic developing solution containing the sugar chain antigen is neutralized at the neutralizing reagent region 6, so that it is adjusted to the neutral range. As a result, the sugar chain antigen is further developed and moved to the downstream region under neutral conditions. In the label region 2, with the development of a specimen sample, a labeled antibody is released into the liquid, and it is then developed to the support 1. When a sugar chain antigen is present in a specimen sample, the sugar chain antigen is specifically captured by a capturing antibody at a detection region 3 in the support 1, and the sugar chain antigen also has a specific reaction with a labeled antibody to form a complex. Thereby, at the detection region 3, the sandwiching of the antibody via the sugar chain antigen is achieved, so that a labeled antibody-sugar chain antigen complex can be measured at the detection region 3. The detection region can be observed through a judgment portion of the immunochromatographic device.

According to the method for using the immunochromatographic test piece, since the extraction of a sugar chain antigen from a specimen is carried out on the immunochromatographic test piece, it is not necessary to previously extract the sugar chain antigen from the specimen before the measurement using the immunochromatographic test piece, but the sugar chain antigen in the specimen can be measured by a single step.

In the immunochromatographic test piece, in which a dried pad containing a solid acid reagent or nitrite is incorporated, efficient extraction can be performed on a reagent.

The specimen addition port may have an area almost equivalent to that of a pad containing a solid acid reagent (a solid acid reagent region). Thereby, a larger amount of sample can be instantly contacted with the solid acid reagent. Furthermore, by adopting a highly hydrophobic non-woven fabric as a member for application or impregnation, a sample cannot be developed to a downstream pad impregnated with a neutralizing reagent (a neutralizing reagent region) immediately after addition of the sample. Thereby, the sample is retained in a specimen addition portion for 1 to 2 minutes, so that an antigen can be sufficiently extracted.

For the immunochromatographic device, when the solid acid reagent region or the nitrite region and the neutralizing reagent region are overlapped and contacted with each other, a sheet impermeable to a liquid is established such that the sheet is sandwiched between the solid acid reagent region or the nitrite region and the neutralizing reagent region. The establishment of the sheet can produce the following three effects.
(A) The movement of a reagent between two adjacent regions can be suppressed during preservation of the test piece. As a result, the reaction of a solid acid reagent or nitrite with a neutralizing reagent by contact can be prevented. As a result, the stability of the reagent can be improved.
(B) When a specimen mixed with nitrite or an acid reagent is added, insufficient extraction of a sugar chain antigen ascribable to the specimen that reaches the solid acid reagent region or the nitrite region and immediately thereafter moves to the neutralizing reagent region can be prevented. That is, the rate at which a liquid containing the specimen moves from the solid acid reagent region or the nitrite region to the neutralizing reagent region is decreased, and the time for which the liquid containing the specimen stays in the solid acid reagent region or the nitrite region is lengthened. As a result, a sugar chain antigen is sufficiently extracted, and measurement sensitivity is enhanced.
(C) When a specimen mixed with nitrite or an acid reagent is added, a liquid containing the specimen that has reached the solid acid reagent region or the nitrite region and immediately thereafter moved to the neutralizing reagent region can be prevented from flowing backward to the solid acid reagent region or the nitrite region, reducing the activity of the solid acid reagent or the nitrite, and reducing extraction efficiency. That is, a liquid containing the specimen that has once reached the neutralizing reagent region is prevented from flowing backward to the solid acid reagent region or the nitrite region and prevented from reducing the activity of the solid acid reagent or the nitrite. Thus, the extraction of the sugar chain antigen with nitrous acid is allowed to proceed efficiently, and measurement sensitivity is enhanced.

The material for the sheet to be established between the solid acid reagent region or the nitrite region and the neutralizing reagent region is not limited as long as the material is impermeable to a liquid. For example, a resin-made sheet such as a PET (polyethylene terephthalate) sheet or a polyethylene sheet can be used. The resin-made sheet is also referred to as a resin-made film.

The sheet may be established such that the solid acid reagent region or the nitrite region and the neutralizing reagent region are partially contacted and overlapped with each other. In this case, the length of the overlapped portion between the solid acid reagent region or the nitrite region and the neutralizing reagent region is preferably minimized and is, for example, 5 mm or less, preferably 3 mm or less, more preferably 2 mm or less.

The sheet may be established so as not to allow the solid acid reagent region or the nitrite region and the neutralizing reagent region into contact with each other, and the pad impregnated with the acid reagent or the nitrite may have a shape overhanging a PET sheet. In this case, the neutralization region may be completely coated with the sheet, and the solid acid reagent region or the nitrous acid region may be established on the sheet. In this case, a liquid within the solid acid reagent region or the nitrous acid region flows on the sheet without moving directly to the neutralization region, and then arrives at the neutralizing reagent region.

The sheet may be established, for example, only between the solid acid reagent region or the nitrite region and the neutralizing reagent region. On the other hand, when the resin-made sheet adheres, as a top laminate sheet, to and covers the upper side of the immunochromatographic test piece, the top laminate sheet adheres to and covers the neutralizing reagent region, the label region, the support, the detection region, and the absorption band excluding the solid acid reagent region or the nitrous acid region and further excluding the sample pad when the solid acid reagent region or the nitrous acid region also serves as the sample pad. The solid acid reagent region or the nitrite region may adhere to the upper part of the top laminate sheet adhering to the upper part of the neutralizing reagent portion.

### Studies regarding size of addition port

In the following examples, % represents w/v% unless otherwise specified.
1. Immobilization of *anti-Streptococcus pyogenes* (group A β-hemolytic streptococcus) antibody on nitrocellulose membrane (support)
A solution obtained by diluting an *anti-Streptococcus pyogenes* antibody with purified water to a concentration of 1.0 mg/mL and an anti-rabbit IgG antibody were prepared. The anti-*Streptococcus pyogenes* antibody was linearly applied to the sample pad side of a nitrocellulose membrane backed with a PET (polyethylene terephthalate) film, and the anti-rabbit IgG antibody was linearly applied to the absorption band side thereof. Thereafter, the nitrocellulose membrane was dried at 45°C for 30 minutes to obtain an *anti-Streptococcus pyogenes* antibody-immobilized membrane. This membrane is referred to as an "antibody-immobilized membrane" in the present example.
2. Immobilization of *anti-Streptococcus pyogenes* antibody on colored polystyrene particles
An *anti-Streptococcus pyogenes* antibody was diluted with purified water to a concentration of 1.0 mg/mL, and colored polystyrene particles were then added to the obtained solution to a concentration of 0.1 %, followed by stirring. Thereafter, carbodiimide was added to the mixed solution to a concentration of 1 %, and the obtained mixture was further stirred. A supernatant was removed from the reaction mixture by a centrifugal operation, and was then re-suspended in 50 mM Tris (pH 9.0) and 3 % BSA to obtain a 0.04 % *anti-Streptococcus pyogenes* antibody-bound colored polystyrene particle suspension. These particles are referred to as "antibody-immobilized particles" in the present example.
3. Application and drying of *anti-Streptococcus pyogenes* antibody-bound colored polystyrene particles
The antibody-immobilized particle suspension produced in the above 2 was applied in a predetermined amount to a non-woven fabric, and was then dried at 45°C for 30 minutes. The obtained non-woven fabric is referred to as a "dry pad" in the present example.
4. Production of neutralizing reagent (basic reagent) pad
3 M Trizma (brand name) Base (Tris base) as a neutralizing reagent (basic reagent), and 1.5 % Triton X-100 were applied in a concentration of 30 µL/cm to a filter (Toyo Roshi Kaisha, Ltd.; No. 26-3).
5. Production of solid acid reagent pad
1.0 M tartaric acid as a solid acid reagent, and 0.5 % Triton X-100 were applied in a concentration of 13 µL/cm to a non-woven fabric (Unitika, Ltd.; Elves). Immediately after the application, the non-woven fabric was dried at 45°C for 1 hour to obtain a solid acid reagent-impregnated non-woven fabric.
6. Production of immunochromatographic test piece for *Streptococcus pyogenes* detection
The antibody-immobilized membrane, the dry pad, and the neutralizing reagent (basic reagent) pad were each adhered to other members (a backing sheet and an absorption band), followed by cutting the resultant into a piece with a width of 5 mm, thereby producing a *Streptococcus pyogenes* test piece. In the present example, a test piece, in which the solid acid reagent- and neutralizing reagent (basic reagent)-impregnated filters are used as a sample pad, are referred to as "the present immunochromatographic test piece." It is to be noted that the immunochromatographic test piece comprises, from a site upstream thereof, a solid acid reagent-impregnated non-woven fabric, a neutralizing reagent (basic reagent)-impregnated non-woven fabric, a dry pad (label region), an antibody-immobilized membrane (detection region), and an absorption band, along the flow of a specimen.
7. Immunochromatographic device
The present immunochromatographic test piece was incorporated into each of a conventional immunochromatographic device having an addition port length of 5 mm and an immunochromatographic device having an addition port length of 10 mm, i. e. a widened addition port with a size to which 10 µL to 200 µL of the specimen sample can be supplied, and the following test was conducted.
8. Specimen
*Streptococcus pyogenes* was cultured, and the culture solution was then adjusted to cell counts of 1.0 × 10⁷ CFU/mL, using a normal saline.
As a negative specimen, a physiological saline was used.
9. Measurement
20 µL of a specimen was suspended in 180 µL of a sodium nitrite solution (2.0 M NaNO₃, 1 % Tween 20), and 75 µL of the suspension was then added to the addition port of the immunochromatographic device. On the other hand, as a conventional method, a specimen was suspended in a nitrous acid extract solution in which sodium nitrite was mixed with hydrochloric acid, and 50 µL of a specimen suspension that had been neutralized with a Tris solution was then added to the addition port of the immunochromatographic device of the conventional method (in which neither the acid reagent nor the neutralizing reagent was immobilized). Five minutes later, the presence or absence of accumulation of colored polystyrene particles in a predetermined position on which an *anti-Streptococcus pyogenes* antibody had been immobilized, and the degree of accumulation, were judged by visual observation. The linear accumulation was judged as +++, ++, and +, in the order of the accumulation strength. When it was hard to judge the accumulation degree, it was judged as ±, and when no accumulation was observed, it was judged as -.
10. Results

**[Table 1]**

| Dropwise addition port length | The number of repeats | 1min | 2min | 3min | 4min | 5min |
|---|---|---|---|---|---|---|
| 5mm | N=1 | - | + | + | ++ | ++ |
| | N=2 | - | + | ± | + | ++ |
| | N=3 | - | + | ± | ++ | ++ |
| 10.5mm | N=1 | + | + | ++ | +++ | +++ |
| | N=2 | + | ++ | ++ | +++ | +++ |
| | N=3 | + | + | ++ | ++ | +++ |

As shown in Table 1, the immunochromatographic device having a larger addition port length of 10 mm had higher measurement sensitivity than that of the immunochromatographic device having an addition port length of 5 mm. This is considered because antigen extraction efficiency by a nitrous acid treatment was enhanced.

### Studies regarding applied amount of surfactant

Test results (Table 2) were comparatively analyzed under conditions where the concentration of the surfactant Triton X-100 for the solid acid reagent. Production of solid acid reagent-impregnated non-woven fabric was 0.5 % or 2.0 %.

**[Table 2]**

| R2 additive | 1min | 2min | 3min | 4min | 5min |
|---|---|---|---|---|---|
| 0.5 %Tx100 | - | - | + | ++ | ++ |
| 2.0 %Tx100 | - | - | ± | + | + |

As seen from the results of Table 2, the lower surfactant concentration resulted in higher measurement sensitivity. This is considered because antigen extraction efficiency by a nitrous acid treatment was enhanced.

### LIST OF REFERENCE SIGNS

- 1: Support (comprising a detection region)
- 2: Label region
- 3: Detection region
- 4: Sample pad
- 5: Solid acid reagent region
- 6: Neutralizing reagent region
- 7: Absorption band
- 8: Backing sheet
- 9: Addition port of a device
- 10: Judgment portion of a device

## Claims

1. An immunochromatographic device comprising an immunochromatographic test piece for extracting and measuring a sugar chain antigen in a specimen, and a container which stores the test piece, the immunochromatographic device having a specimen addition port in a sample pad of the test piece, the immunochromatographic test piece comprising: a sample pad to which a specimen mixed with nitrite or an acid solution is added; a label region comprising a labeled antibody obtained by labeling an antibody against the sugar chain antigen; and a detection region on which the antibody against the sugar chain antigen is immobilized, wherein an antibody-sugar chain antigen-labeled antibody complex is formed in the detection region to measure the sugar chain antigen, and the immunochromatographic test piece having a region impregnated with a neutralizing reagent upstream of the label region, and further having a region impregnated with a solid acid reagent when the specimen mixed with the nitrite is used, or a region impregnated with nitrite when the specimen mixed with the acid solution is used, upstream of the region impregnated with the neutralizing reagent, wherein
the immunochromatographic device
(i) has a specimen addition port for promoting the extraction of the sugar chain antigen with the nitrite and the solid acid reagent by retaining an added specimen sample solution and supplying the specimen sample solution in a short time to the region impregnated with the solid acid reagent or the nitrite, wherein the specimen addition port of the immunochromatographic device has a size to which 10 *µ* L to 200 *µ* L of the specimen sample can be supplied, and
(ii) has no space between the addition port and the sample pad so as to prevent the sample from escaping from the addition port.

2. The immunochromatographic device according to claim 1, wherein the pad impregnated with the solid acid reagent or the nitrite, or the neutralizing reagent on the immunochromatographic test piece is made of a highly hydrophobic material, and the extraction of the sugar chain antigen with the nitrite and the solid acid reagent is promoted by slowing a development time of the specimen sample solution.

3. The immunochromatographic device according to claim 2, wherein the highly hydrophobic material is selected from the group consisting of polyethylene, polyester, polystyrene, polypropylene, rayon, and nylon.

4. The immunochromatographic device according to claim 1, wherein the pad impregnated with the solid acid reagent or the nitrite, or the neutralizing reagent on the immunochromatographic test piece is made of a material having a high degree of hydrophilicity, and the extraction of the sugar chain antigen with the nitrite and the solid acid reagent is promoted by slowing a development time of the specimen sample solution.

5. The immunochromatographic device according to claim 4, wherein the material having a high degree of hydrophilicity is a thick filter.

6. The immunochromatographic device according to any one of claims 1 to 5, wherein the extraction of the sugar chain antigen with the nitrite and the solid acid reagent is promoted by adding a surfactant to the pad impregnated with the solid acid reagent or the nitrite, or the neutralizing reagent on the immunochromatographic test piece, and adjusting the development time of the specimen sample solution.

7. The immunochromatographic device according to claim 6, wherein the surfactant is selected from the group consisting of polyoxyethylene octyl phenyl ether, polyoxyethylene sorbitan fatty acid ester, polyoxyethylene alkyl ether, sodium cholate, deoxycholic acid, sodium lauryl sulfate, and sodium dodecylbenzenesulfonate.

8. The immunochromatographic device according to any one of claims 1 to 7, wherein the extraction of the sugar chain antigen with the nitrite and the solid acid reagent is promoted by adding a high-molecular-weight compound having immobilizing action to the pad impregnated with the solid acid reagent or the nitrite, or the neutralizing reagent on the immunochromatographic test piece, and adjusting the development time of the specimen sample solution.

9. The immunochromatographic device according to claim 8, wherein the high-molecular-weight compound having immobilizing action is PVA or PLL.

10. The immunochromatographic device according to any one of claims 1 to 9, wherein the region impregnated with the solid acid reagent or the nitrite is present on the sample pad or on the pad with the label region applied thereto.

11. The immunochromatographic device according to any one of claims 1 to 10, wherein the solid acid reagent is selected from the group consisting of malonic acid, malic acid, maleic acid, citric acid, and tartaric acid.

12. The immunochromatographic device according to any one of claims 1 to 11, wherein the neutralizing reagent is tris(hydroxymethyl)aminomethane or sodium hydroxide.

13. The immunochromatographic device according to any one of claims 1 to 12, wherein the sugar chain antigen is the sugar chain antigen of protozoa, fungi, bacteria, mycoplasma, rickettsia, chlamydia, or virus.

14. A method of measuring a sugar chain antigen in a specimen according to an immunochromatography method using the immunochromatographic device according to any one of claims 1 to 13,
the method measuring the sugar chain antigen in the specimen by promoting the extraction of the sugar chain antigen according to the immunochromatography method, and the method comprising mixing the specimen with a nitrous acid solution when the immunochromatographic device has a region impregnated with a solid acid reagent, or mixing the specimen with an acid solution when the immunochromatographic device has a region impregnated with nitrite, and adding the mixture to a sample pad of the immunochromatographic device, wherein in the immunochromatography method,
the sugar chain antigen is extracted from the specimen by the action of nitrous acid generated through a reaction of the nitrite with the solid acid reagent in the region impregnated with the solid acid reagent or the region impregnated with the nitrite, the acid solution comprising the sugar chain antigen is neutralized in a region impregnated with a neutralizing reagent, and
an antibody-sugar chain antigen-labeled antibody complex is formed in a detection region.

## Patentansprüche

1. Immunochromatografische Vorrichtung, die ein immunochromatografisches Teststück zur Extraktion und Messung eines Zuckerkettenantigens in einer Probe und einen Behälter zur Aufbewahrung des Teststücks aufweist, wobei die immunochromatografische Vorrichtung eine Probenzugabeöffnung in einem Probenfeld des Teststücks aufweist, wobei das immunochromatografische Teststück aufweist: ein Probenfeld, zu dem eine mit Nitrit oder einer Säurelösung gemischte Probe zugegeben wird; einen Markierungsbereich, der einen markierten Antikörper aufweist, der durch Markieren eines Antikörpers gegen das Zuckerkettenantigen erhalten wurde; und einen Nachweisbereich, auf dem der Antikörper gegen das Zuckerkettenantigen immobilisiert ist, wobei ein Antikörper-Zuckerkettenantigen-markierter Antikörperkomplex im Nachweisbereich gebildet ist, um das Zuckerkettenantigen zu messen, und wobei das immunochromatografische Teststück einen mit einem Neutralisierungsreagenz imprägnierten Bereich stromaufwärts des Markierungsbereichs aufweist, und weiterhin stromaufwärts des mit dem Neutralisierungsreagenz imprägnierten Bereichs einen mit einem festen Säurereagenz imprägnierten Bereich, wenn die mit dem Nitrit gemischte Probe verwendet wird, oder einen mit Nitrit imprägnierten Bereich, wenn die mit der Säurelösung gemischte Probe verwendet wird, aufweist, wobei
die immunochromatografische Vorrichtung
(i) eine Probenzugabeöffnung aufweist, um die Extraktion des Zuckerkettenantigens mit dem Nitrit und dem festen Säurereagenz zu unterstützen, indem eine zugegebene Probenlösung zurückgehalten wird und die Probenlösung in kurzer Zeit dem mit dem festen Säurereagenz oder dem Nitrit imprägnierten Bereich zugeführt wird, wobei die Probenzugabeöffnung der immunochromatografischen Vorrichtung eine Größe aufweist, der 10 µL bis 200 µL der Probe zugeführt werden können, und
(ii) keinen Abstand zwischen der Zugabeöffnung und dem Probenfeld aufweist, so dass die Probe nicht aus der Zugabeöffnung entweichen kann.

2. Immunochromatografische Vorrichtung nach Anspruch 1, wobei das mit dem festen Säurereagenz oder dem Nitrit oder dem Neutralisierungsreagenz imprägnierte Feld auf dem immunochromatografischen Teststück aus einem stark hydrophoben Material hergestellt ist und die Extraktion des Zuckerkettenantigens mit dem Nitrit und dem festen Säurereagenz durch Verlangsamung einer Entwicklungszeit der Probenlösung gefördert wird.

3. Immunochromatografische Vorrichtung nach Anspruch 2, wobei das stark hydrophobe Material ausgewählt ist aus der Gruppe bestehend aus Polyethylen, Polyester, Polystyrol, Polypropylen, Rayon und Nylon.

4. Immunochromatografische Vorrichtung nach Anspruch 1, wobei das mit dem festen Säurereagenz oder dem Nitrit oder dem Neutralisierungsreagenz imprägnierte Feld auf dem immunochromatografischen Teststück aus einem Material mit einem hohen Grad an Hydrophilie hergestellt ist und die Extraktion des Zuckerkettenantigens mit dem Nitrit und dem festen Säurereagenz durch Verlangsamung der Entwicklungszeit der Probenlösung gefördert wird.

5. Immunochromatografische Vorrichtung nach Anspruch 4, wobei das Material mit einem hohen Grad an Hydrophilie ein dicker Filter ist.

6. Immunochromatografische Vorrichtung nach einem der Ansprüche 1 bis 5, wobei die Extraktion des Zuckerkettenantigens mit dem Nitrit und dem festen Säurereagenz durch Zugabe eines Tensids zu dem mit dem festen Säurereagenz oder dem Nitrit oder dem Neutralisierungsreagenz imprägnierten Feld auf dem immunochromatografischen Teststück und durch Anpassen der Entwicklungszeit der Probenlösung gefördert wird.

7. Immunochromatografische Vorrichtung nach Anspruch 6, wobei das Tensid ausgewählt ist aus der Gruppe bestehend aus Polyoxyethylenoctylphenylether, Polyoxyethylensorbitanfettsäureester, Polyoxyethylenalkylether, Natriumcholat, Desoxycholsäure, Natriumlaurylsulfat und Natriumdodecylbenzolsulfonat.

8. Immunochromatografische Vorrichtung nach einem der Ansprüche 1 bis 7, wobei die Extraktion des Zuckerkettenantigens mit dem Nitrit und dem festen Säurereagenz durch Zugabe einer hochmolekularen Verbindung mit immobilisierender Wirkung zu dem mit dem festen Säurereagenz oder dem Nitrit oder dem Neutralisierungsreagenz imprägnierten Feld auf dem immunochromatografischen Teststück und durch Anpassen der Entwicklungszeit der Probenlösung gefördert wird.

9. Immunochromatografische Vorrichtung nach Anspruch 8, wobei die hochmolekulare Verbindung mit immobilisierender Wirkung PVA oder PLL ist.

10. Immunochromatografische Vorrichtung nach einem der Ansprüche 1 bis 9, wobei der mit dem festen Säurereagenz oder dem Nitrit imprägnierte Bereich auf dem Probenfeld oder auf dem Feld mit dem darauf aufgebrachten Markierungsbereich vorliegt.

11. Immunochromatografische Vorrichtung nach einem der Ansprüche 1 bis 10, wobei das feste Säurereagenz ausgewählt ist aus der Gruppe bestehend aus Malonsäure, Äpfelsäure, Maleinsäure, Zitronensäure und Weinsäure.

12. Immunochromatografische Vorrichtung nach einem der Ansprüche 1 bis 11, wobei das Neutralisierungsreagenz Tris(hydroxymethyl)aminomethan oder Natriumhydroxid ist.

13. Immunochromatografische Vorrichtung nach einem der Ansprüche 1 bis 12, wobei das Zuckerkettenantigen das Zuckerkettenantigen von Protozoen, Pilzen, Bakterien, Mykoplasmen, Rickettsien, Chlamydien oder Viren ist.

14. Verfahren zur Messung eines Zuckerkettenantigens in einer Probe nach einem immunochromatografischen Verfahren unter Verwendung der immunochromatografischen Vorrichtung nach einem der Ansprüche 1 bis 13,
wobei das Verfahren das Zuckerkettenantigen in der Probe durch Förderung der Extraktion des Zuckerkettenantigens gemäß dem immunochromatografischen Verfahren misst, und das Verfahren
ein Mischen der Probe mit einer Salpetersäurelösung, wenn die immunochromatografische Vorrichtung einen Bereich aufweist, der mit einem festen Säurereagenz imprägniert ist, oder ein Mischen der Probe mit einer Säurelösung, wenn die immunochromatografische Vorrichtung einen Bereich aufweist, der mit Nitrit imprägniert ist, und ein Hinzufügen der Mischung zu einem Probenfeld der immunochromatografischen Vorrichtung umfasst, wobei in dem immunochromatografischen Verfahren
das Zuckerkettenantigen aus der Probe durch die Wirkung von Salpetersäure extrahiert wird, die durch eine Reaktion des Nitrits mit dem festen Säurereagenz in dem mit dem festen Säurereagenz imprägnierten Bereich oder dem mit dem Nitrit imprägnierten Bereich erzeugt wird, die Säurelösung, die das Zuckerkettenantigen aufweist, in einem mit einem Neutralisierungsreagenz imprägnierten Bereich neutralisiert wird, und
ein Antikörper-Zuckerketten-Antigen-markierter Antikörperkomplex in einer Nachweisregion gebildet wird.

## Revendications

1. Dispositif immunochromatographique comprenant une pièce de test immunochromatographique pour extraire et mesurer un antigène de chaîne de sucre dans un spécimen, et un récipient qui stocke la pièce de test, le dispositif immunochromatographique ayant un port d'ajout de spécimen dans un champ d'échantillon de la pièce de test, la pièce de test immunochromatographique comprenant : un champ d'échantillon auquel est ajouté un spécimen mélangé à du nitrite ou à une solution acide ; une zone de marquage comprenant un anticorps marqué obtenu en marquant un anticorps contre l'antigène de chaîne de sucre ; et une zone de détection sur laquelle l'anticorps contre l'antigène de chaîne de sucre est immobilisé, dans laquelle un complexe anticorps-antigène de chaîne de sucre-anticorps marqué est formé dans la zone de détection pour mesurer l'antigène de chaîne de sucre, et la pièce de test immunochromatographique ayant une zone imprégnée d'un réactif de neutralisation en amont de la zone de marquage, et ayant en outre une zone imprégnée d'un réactif acide solide lorsque le spécimen mélangé au nitrite est utilisé, ou une zone imprégnée de nitrite lorsque le spécimen mélangé à la solution acide est utilisé, en amont de la zone imprégnée du réactif de neutralisation, dans lequel
le dispositif immunochromatographique
(i) présente un port d'ajout de spécimen pour favoriser l'extraction de l'antigène de chaîne de sucre avec le nitrite et le réactif acide solide en retenant une solution échantillon de spécimen ajoutée et en fournissant la solution échantillon de spécimen à la zone imprégnée du réactif acide solide ou du nitrite en peu de temps, dans lequel le port d'ajout de spécimen du dispositif immunochromatographique a une taille à laquelle 10 µl à 200 µl de l'échantillon de spécimen peuvent être fourni, et
(ii) n'a pas d'espace entre le port d'ajout et le champ d'échantillon de manière à empêcher l'échantillon de s'échapper du port d'ajout.

2. Dispositif immunochromatographique selon la revendication 1, dans lequel le champ imprégné du réactif acide solide ou du nitrite, ou du réactif de neutralisation sur la pièce de test immunochromatographique est fait d'un matériau hautement hydrophobe, et l'extraction de l'antigène de chaîne de sucre avec le nitrite et le réactif acide solide est favorisée en ralentissant un temps de développement de la solution échantillon de spécimen.

3. Dispositif immunochromatographique selon la revendication 2, dans lequel le matériau hautement hydrophobe est choisi dans le groupe constitué par le polyéthylène, le polyester, le polystyrène, le polypropylène, la rayonne et le nylon.

4. Dispositif immunochromatographique selon la revendication 1, dans lequel le champ imprégné du réactif acide solide ou du nitrite, ou du réactif de neutralisation sur la pièce de test immunochromatographique est fait d'un matériau ayant un degré élevé d'hydrophilie, et l'extraction de l'antigène de chaîne de sucre avec le nitrite et le réactif acide solide est favorisée en ralentissant le temps de développement de la solution échantillon de spécimen.

5. Dispositif immunochromatographique selon la revendication 4, dans lequel le matériau à haut degré d'hydrophilie est un filtre épais.

6. Dispositif immunochromatographique selon l'une quelconque des revendications 1 à 5, dans lequel l'extraction de l'antigène de chaîne de sucre avec le nitrite et le réactif acide solide est favorisée en ajoutant un tensioactif au champ imprégné du réactif acide solide ou du nitrite, ou du réactif de neutralisation sur la pièce de test immunochromatographique, et en ajustant le temps de développement de la solution échantillon de spécimen.

7. Dispositif immunochromatographique selon la revendication 6, dans lequel le tensioactif est choisi dans le groupe constitué de l'éther de polyoxyéthylène octyle phényle, de l'ester d'acide gras de polyoxyéthylène sorbitane, de l'éther d'alkyle de polyoxyéthylène, du cholate de sodium, de l'acide désoxycholique, du laurylsulfate de sodium, et du dodécylbenzènesulfonate de sodium.

8. Dispositif immunochromatographique selon l'une quelconque des revendications 1 à 7, dans lequel l'extraction de l'antigène de chaîne de sucre avec le nitrite et le réactif acide solide est favorisée en ajoutant un composé de poids moléculaire élevé ayant une action immobilisante sur le champ imprégné du réactif acide solide ou du nitrite, ou du réactif de neutralisation sur la pièce de test immunochromatographique, et en ajustant le temps de développement de la solution échantillon de spécimen.

9. Dispositif immunochromatographique selon la revendication 8, dans lequel le composé de poids moléculaire élevé ayant une action immobilisante est le PVA ou le PLL.

10. Dispositif immunochromatographique selon l'une quelconque des revendications 1 à 9, dans lequel la zone imprégnée du réactif acide solide ou du nitrite est présente sur le champ d'échantillon ou sur le champ avec la zone de marquage appliquée sur celui-ci.

11. Dispositif immunochromatographique selon l'une quelconque des revendications 1 à 10, dans lequel le réactif acide solide est choisi dans le groupe constitué de l'acide malonique, de l'acide malique, de l'acide maléique, de l'acide citrique et de l'acide tartrique.

12. Dispositif immunochromatographique selon l'une quelconque des revendications 1 à 11, dans lequel le réactif de neutralisation est le tris(hydroxyméthyl)aminométhane ou l'hydroxyde de sodium.

13. Dispositif immunochromatographique selon l'une quelconque des revendications 1 à 12, dans lequel l'antigène de chaîne de sucre est l'antigène de la chaîne de sucre d'un protozoaire, d'un champignon, d'une bactérie, d'un mycoplasme, d'une rickettsie, d'une chlamydia ou d'un virus.

14. Méthode de mesure d'un antigène de chaîne de sucre dans un spécimen selon une méthode d'immunochromatographie utilisant le dispositif immunochromatographique selon l'une quelconque des revendications 1 à 13,
la méthode mesurant l'antigène de chaîne de sucre dans le spécimen en favorisant l'extraction de l'antigène de chaîne de sucre selon la méthode d'immunochromatographie, et la méthode comprenant
mélanger le spécimen avec une solution d'acide nitreux lorsque le dispositif immunochromatographique présente une zone imprégnée d'un réactif acide solide, ou mélanger le spécimen avec une solution acide lorsque le dispositif immunochromatographique présente une zone imprégnée de nitrite, et ajouter le mélange à un champ d'échantillon du dispositif immunochromatographique, dans lequel, dans la méthode d'immunochromatographie,
l'antigène de chaîne de sucre est extrait du spécimen par l'action de l'acide nitreux généré par une réaction du nitrite avec le réactif acide solide dans la zone imprégnée du réactif acide solide ou la zone imprégnée du nitrite, la solution acide comprenant l'antigène de chaîne de sucre est neutralisée dans une zone imprégnée d'un réactif de neutralisation, et
un complexe anticorps-antigène de chaîne de sucre-anticorps marqué est formé dans une zone de détection.
